# DEMANDE DE BREVET EUROPEEN

(11) **EP 4 008 692 A1**
(43) Date de publication de la demande: **08.06.2022**
(21) Numéro de dépôt: 20306487.8
(22) Date de dépôt: 03.12.2020
(51) Int. Cl.: C02F 11/04, C02F 11/18, C12M 1/107, C12P 5/02, C02F 9/00, C10J 1/00, C12M 1/00, C02F 11/10

(54) **PROCEDE ET SYSTEME DE PRODUCTION DE BIOGAZ ET DE TRAITEMENT DE BOUES DE STATION D'EPURATION**

(71) Demandeur: Syctom L'Agence Metropolitaine Des Dechets Menagers, 75001 Paris (FR); Syndicat Interdepartemental pour l'Assainissement de l'Agglomeration Parisienne, 75012 Paris (FR)
(72) Inventeur: AUMAITRE, Vincent, 75005 Paris (FR); BOHN, Michael, 70569 Stuttgart (DE); DALVERNY, Cécile, 10829 Berlin (DE)
(74) Mandataire: Plasseraud IP

(57) **Abrégé**

La présente invention concerne un procédé de production de biogaz et de traitement d'au moins un intrant comprenant de la matière organique, notamment de boues de station d'épuration et/ou de la fraction organique récupérable d'ordures ménagères, comprenant les étapes suivantes :
- digérer de manière anaérobie au moins un intrant comprenant de la matière organique, notamment des boues de station d'épuration et/ou de la fraction organique récupérable d'ordures ménagères, pour produire du méthane et un digestat ;
- réaliser une carbonisation hydrothermale du digestat pour produire de l'hydrochar et de l'eau de process ;
caractérisé en ce que :
dans une étape additionnelle, du méthane est généré à partir de la gazéification de l'hydrochar.

## Description

### Domaine de l'invention

La présente invention concerne un procédé et un système de production de biogaz et de traitement de boues de station d'épuration et/ou de la fraction organique récupérable d'ordures ménagères.

### Arrière-plan technique

La production de biogaz, notamment de méthane, par digestion anaérobie de substrats organiques connaît actuellement un développement soutenu dans le cadre de la lutte contre le réchauffement climatique. Le biogaz ainsi produit est en effet une source d'énergie renouvelable susceptible de remplacer les combustibles fossiles.

L'intérêt de la digestion anaérobie est double puisque ce procédé permet également de traiter des déchets contenant de la matière organique, tels que les boues de stations d'épuration.

Bien qu'avantageux, ce type de procédé de production de biogaz et de traitement de boues de station d'épuration peut cependant être amélioré, notamment en ce qui concerne le rendement de production de méthane, car la digestion anaérobie est incomplète, dans la mesure où le digestat contient généralement encore de la matière organique.

Ainsi, l'article Parmar & Ross (2019) "Integration of Hydrothermal Carbonisation with Anaerobic Digestion; Opportunities for Valorisation of Digestate" Energies 12:1586 expérimente le traitement par carbonisation hydrothermale (HTC) de quatre digestats différents issus d'une digestion anaérobie (AD), incluant les boues d'épuration. La HTC donne de l'eau de process et un hydrochar. Il est suggéré de valoriser l'hydrochar, mal adapté à la combustion, en l'utilisant pour amender les sols. L'eau de process de la HTC est quant à elle soumise à une digestion anaérobie secondaire pour donner du méthane. Cette digestion secondaire permet ainsi d'améliorer le rendement de production de méthane par rapport à une digestion anaérobie simple.

Toutefois, il serait intéressant de pouvoir encore optimiser le rendement en méthane des procédés de traitement de boues d'épuration par digestion anaérobie.

### Résumé de l'invention

La présente invention découle de la mise en évidence inattendue, par les inventeurs, que l'hydrochar issu de la carbonisation hydrothermale de digestats anaérobies de boues de stations d'épuration pouvait être utilisé pour produire du méthane et améliorer le rendement des procédés de production de biogaz.

La présente invention concerne ainsi un procédé de production de biogaz et de traitement d'au moins un intrant comprenant de la matière organique, notamment de boues de station d'épuration et/ou de la fraction organique récupérable d'ordures ménagères, comprenant les étapes suivantes :
- digérer de manière anaérobie au moins un intrant comprenant de la matière organique, notamment des boues de station d'épuration et/ou de la fraction organique récupérable d'ordures ménagères, pour produire du méthane et un digestat ;
- réaliser une carbonisation hydrothermale du digestat pour produire de l'hydrochar et de l'eau de process ;
caractérisé en ce que :
dans une étape additionnelle, du méthane est généré à partir de la gazéification de l'hydrochar.

La présente invention concerne également un système de production de biogaz et de traitement d'au moins un intrant comprenant de la matière organique, notamment de boues de station d'épuration et/ou de la fraction organique récupérable d'ordures ménagères, comprenant :
- un digesteur anaérobie, alimenté par au moins un intrant comprenant de la matière organique, notamment des boues de station d'épuration et/ou de la fraction organique récupérable d'ordures ménagères, qui produit du méthane et un digestat ;
- un module de carbonisation hydrothermale qui produit un hydrochar et de l'eau de process à partir du digestat ;
caractérisé en ce que:
le système comprend en outre des moyens pour générer du méthane à partir de l'hydrochar.

### Description de la Figure

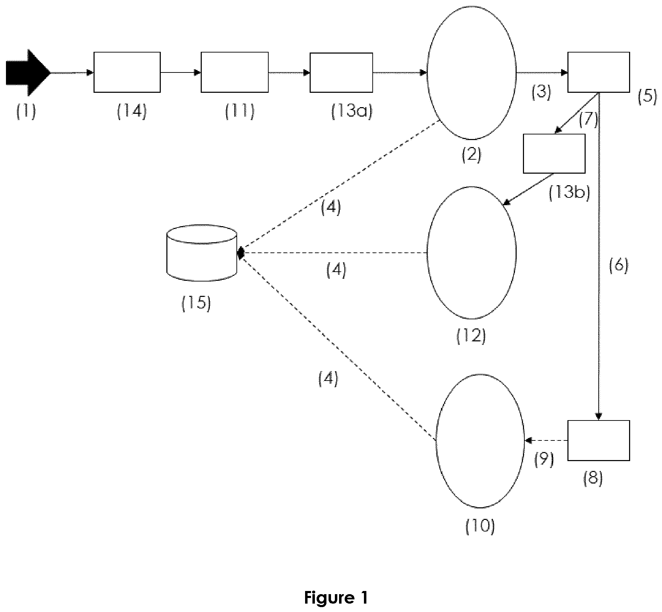

La figure 1 représente un procédé/système de production de biogaz selon l'invention.

### Description de l'invention

A titre préliminaire, on rappellera que le terme « comprenant » signifie « incluant», « contenant » ou « englobant », c'est-à-dire que lorsqu'un objet « comprend » un élément ou plusieurs éléments, d'autres éléments que ceux mentionnés peuvent également être compris dans l'objet. A contrario, l'expression « consistant en » signifie « constitué de », c'est-à-dire que lorsqu'un objet « consiste en » un élément ou plusieurs éléments, l'objet ne peut pas comprendre d'autres éléments que ceux mentionnés.

### Biogaz

Comme on l'entend ici, un biogaz est le gaz produit par la fermentation, ou digestion, de matières organiques en l'absence d'oxygène, c'est-à-dire en conditions anaérobies. De préférence, le biogaz selon l'invention est du méthane (CH₄), également nommé biométhane. La production de méthane par digestion anaérobie d'intrants à base de matière organique est également appelée méthanisation.

### Intrant

L'intrant, qui alimente le système ou le procédé selon l'invention, et qui est traité selon l'invention, peut-être de tout type susceptible de subir une digestion anaérobie. Autrement dit, l'intrant comprend de la matière organique, dont au moins une partie est méthanisable. Il peut notamment s'agir d'une ressource organique, de produits issus de l'agriculture, de l'industrie agro-alimentaire, du traitement des eaux usées ou de déchets ménagers, par exemple des déchets alimentaires ou des déchets verts.

De préférence, l'intrant comprend, ou est constitué de, boues de station d'épuration et/ou de la fraction organique récupérable d'ordures ménagères, et peut notamment être un mélange de boues de station d'épuration et de la fraction organique récupérable d'ordure ménagère.

Comme on l'entend ici la «fraction organique récupérable d'ordure ménagère» est considérée comme équivalente à la « fraction fermentescible des ordures ménagères».

Plus préférablement, on digère avec le procédé de l'invention, ou le système selon l'invention est alimenté, à la fois par des boues de station d'épuration et de la fraction organique récupérable d'ordures ménagères, notamment par un mélange de ceux-ci.

Par ailleurs, l'intrant selon l'invention peut également comprendre, ou être constitué, de graisses, végétales ou animales, de fumier, ou de lisier.

### Digesteur

La digestion anaérobie est conduite dans un réacteur également appelé digesteur ou méthaniseur. La digestion anaérobie et les digesteurs anaérobies sont bien connus de la personne du métier. Brièvement, digestion anaérobie résulte de l'action de différents microorganismes constituant un réseau trophique. On distingue classiquement quatre phases successives : l'hydrolyse, l'acidogenèse, l'acétogenèse et la méthanogenèse. C'est lors de la phase de méthanogenèse que le méthane est produit par l'action d'archéobactéries méthanogènes, qui sont des bactéries anaérobies strictes.

De préférence, la digestion anaérobie selon l'invention, notamment la première digestion anaérobie, ou digestion anaérobie principale, du procédé ou du système selon l'invention, est conduite est conduite à une température de 35°C à 42°C pour une digestion mésophile ou de 55°C à 65°C pour une digestion thermophile, sous une pression de 0 à 50 mbar, pendant une durée de 10 à 30 jours, et à un pH de 6,5 à 8. Avantageusement, ces conditions sont particulièrement adaptées à la digestion anaérobie de boues de station d'épuration et/ou de la fraction organique récupérable d'ordures ménagères. D'autres conditions avantageuses de digestion anaérobie, impliquant notamment la température, la pression, la durée et le pH, peuvent être déterminées de manière routinière par la personne du métier à partir des valeurs ci-dessus.

Outre le biogaz, notamment le méthane produit par la digestion anaérobie, celle-ci produit également un digestat.

Différents types de digesteurs anaérobies sont connus de la personne du métier qui peuvent tous être mis en œuvre dans l'invention.

Toutefois, on préfère, selon l'invention, que la digestion anaérobie, notamment la première digestion anaérobie, ou digestion anaérobie principale, du procédé ou du système selon l'invention, soit effectuée par un digesteur anaérobie sélectionné dans le groupe constitué d'un digesteur à piston, d'un digesteur par percolation et d'un digesteur à haute charge.

On préfère tout particulièrement, selon l'invention, que la digestion anaérobie, notamment la première digestion anaérobie, ou digestion anaérobie principale, du procédé ou du système selon l'invention, soit effectuée par un digesteur à piston. De manière avantageuse, le digesteur anaérobie à piston est particulièrement adapté à la digestion anaérobie d'un mélange de boues de station d'épuration et de la fraction organique récupérable d'ordures ménagères et permet d'optimiser la production de biogaz, notamment de méthane, à partir de ce mélange.

### Carbonisation hydrothermale

La carbonisation hydrothermale, souvent abrégée HTC, est bien connue de la personne du métier. Brièvement, elle permet de transformer de la matière organique, notamment le digestat, en un produit dit « hydrocharbon », ou encore « hydrochar», via un processus thermochimique entretenu dans des conditions anoxiques. Plus précisément, la carbonisation hydrothermale est généralement conduite à une température élevée, notamment à une température de 180°C à 260°C, sous une pression élevée, notamment à une pression de 1 à 6 MPa, pendant une durée variable, notamment de 5 min. à 12h. Dans ces conditions, la biomasse perd spontanément son eau, nommée « eau de process », et se transforme en hydrochar par une réaction exothermique qui s'auto-entretient une fois initiée.

De préférence, la carbonisation hydrothermale selon l'invention est conduite à une température de 180°C à 210°C, sous une pression de 1 à 2 MPa et pendant une durée de 30 à 60 min. Avantageusement, ces conditions sont particulièrement adaptées à la production d'un hydrochar à partir d'un digestat anaérobie de boues de station d'épuration et/ou de la fraction organique récupérable d'ordures ménagères, ayant une bonne capacité de génération de méthane.

### Génération de méthane à partir de l'hydrochar

L'hydrochar selon l'invention est utilisé pour produire du méthane.

Les moyens, ou l'étape, pour générer du méthane à partir de l'hydrochar comprennent au moins un module, ou une sous-étape, de gazéification pour produire un syngaz comprenant du dioxyde de carbone (CO₂) et/ou du monoxyde de carbone et du dihydrogène (H₂) à partir de l'hydrochar et un module, ou une sous-étape, de méthanation pour produire du méthane à partir du dioxyde de carbone et/ou monoxyde de carbone et du dihydrogène du syngaz.

La gazéification est un procédé de production d'un combustible gazeux, ici le dihydrogène, par réaction thermochimique à haute température, notamment localement plus de 1000°C, entre un combustible solide ou liquide, ici l'hydrochar, et un partenaire de réaction gazeux, l'agent de gazéification. Les agents de gazéification typiques sont l'oxygène, l'air et la vapeur d'eau. De préférence, la gazéification selon l'invention est une gazéification à l'oxygène (O₂).

Avantageusement, l'utilisation de l'oxygène comme agent de gazéification permet la production d'un gaz de synthèse à faible teneur en azote et une synthèse de méthane sans besoin de séparation azote/méthane a posteriori. Grâce à l'utilisation d'oxygène, il est également possible d'obtenir un gaz brut à faible teneur en goudron, ce qui limite les frais d'épuration.

La gazéification est mise en œuvre dans un réacteur également nommé gazéifieur. Les gazéifieurs sont bien connus de la personne du métier. De préférence, la gazéification selon l'invention est conduite à une température de 900°C à 1100°C. Avantageusement, ces conditions sont optimisées pour le rendement de production de syngaz à partir d'hydrochar.

De préférence, l'hydrochar selon l'invention est séché préalablement à la gazéification. Le séchage de l'hydrochar permet d'améliorer le rendement de production de syngaz.

De préférence également, l'hydrochar selon l'invention est fractionné préalablement à la gazéification, notamment par broyage, en particules ayant un diamètre moyen inférieur à environ 1 mm

Le résultat de la gazéification est un gaz de synthèse, également nommé syngaz. Le syngaz selon l'invention comprend du dioxyde de carbone (CO₂) et/ou du monoxyde de carbone (CO) et du dihydrogène (H₂). Le syngaz selon l'invention peut également comprendre de la vapeur d'eau et de l'azote (N₂).

Le syngaz selon l'invention subit ensuite une méthanation. La méthanation, bien connue de la personne du métier, est une réaction de synthèse du méthane (CH₄) à partir de dihydrogène (H₂) et de monoxyde de carbone (CO) ou de dioxyde de carbone (CO₂). La méthanation peut être chimique ou biologique.

De préférence, la méthanation selon l'invention est biologique, c'est-à-dire que l'hydrogène et le CO₂ contenus dans le gaz sont convertis en méthane et en eau par des microorganismes, notamment hydrogénotrophiques. De préférence, la méthanation selon l'invention est conduite à une température de 35°C à 42°C pour une méthanation mésophile ou de 55°C à 65°C pour une méthanation thermophile, sous une pression de 0 à 5 bar et pendant une durée de 0,5h à 2h.

Avantageusement, outre l'amélioration du rendement de production de méthane, la méthanation permet d'améliorer le bilan carbone du procédé/système de l'invention en captant du dioxyde de carbone (CO₂).

De préférence, l'eau qui est produite au cours de la méthanation est dirigée vers un digesteur anaérobie, notamment vers le digesteur anaérobie qui traite l'eau de process de la carbonisation hydrothermale lorsqu'il est présent. Avantageusement, cela permet d'améliorer le rendement de production de méthane du procédé ou du système selon l'invention car l'eau contient de la matière organique en suspension et des composants du gaz de synthèse qui ont été dissous ou adsorbés.

De préférence, préalablement à la méthanation, le syngaz selon l'invention est soumis à une réaction du gaz à l'eau, également nommée « *water gas shift reaction* » ou encore « *water-shift réaction* ». Avantageusement, cette réaction permet de convertir le monoxyde de carbone et la vapeur d'eau en H₂ et CO₂ ce qui augmente le rendement de production de méthane de la méthanation.

### Etapes/modules additionnels

De préférence, le procédé ou le système selon l'invention comprenant également une étape/un module d'hydrolyse thermique préalable à la digestion anaérobie.

L'hydrolyse thermique est une technique de détente brusque, également nommée « *flashing explosion* » ou « *steam explosion* », bien connue de la personne du métier. Brièvement, pour l'hydrolyse thermique, les intrants sont typiquement chauffés à une température de l'ordre de 165°C, ce qui provoque une surpression de l'ordre de 10 bars induite qui est ensuite diminuée par une détente brusque. Le résultat de l'hydrolyse thermique est un hydrolysat.

Avantageusement, le traitement des intrants par hydrolyse thermique permet une réduction du temps passé dans le digesteur anaérobie ou du volume de celui-ci avec une augmentation simultanée du rendement de production de biogaz, ainsi qu'une amélioration du drainage et une réduction du volume de digestat.

De préférence, l'hydrolyse thermique est appliquée aux boues de station d'épuration et éventuellement à au moins une partie, voire la totalité, de la fraction organique récupérable des ordures ménagères.

De préférence, préalablement à l'hydrolyse thermique les intrants, notamment les boues de station d'épuration, sont épaissis, en particulier en évaporant l'eau qu'ils contiennent par chauffage. L'épaississement, c'est-à-dire l'augmentation de la teneur en matières solides de l'intrant, notamment des boues de station d'épuration, réduit la proportion spécifique d'eau, qui n'a alors pas besoin d'être chauffée et refroidie, ce qui améliore le rendement énergétique global du procédé ou du système selon l'invention.

De préférence, le procédé ou le système selon l'invention comprend également une étape/module de digestion anaérobie de l'eau de process pour produire du méthane. Plus préférablement, la digestion anaérobie de l'eau de process est conduite dans un deuxième digesteur anaérobie, ou digesteur anaérobie secondaire. Avantageusement, cette étape/module de digestion anaérobie secondaire permet d'augmenter le rendement de production de biogaz, notamment de méthane, du procédé ou du système selon l'invention.

La digestion anaérobie de l'eau de process dans le deuxième digesteur anaérobie peut être conduite en mélange avec au moins intrant tel que défini ci-dessus, notamment lorsque l'eau de process est trop pauvre en matière organique. Dans ce cas, le procédé ou le système selon l'invention comprend une étape/module de mélange de l'eau de process avec au moins un intrant contenant de la matière organique.

De préférence, le procédé ou le système selon l'invention comprend également au moins une étape/module de récupération de phosphore et/ou d'ammonium.

De préférence, le procédé ou le système selon l'invention comprend au moins au moins une étape/module de récupération de phosphore préalablement à la digestion anaérobie principale, notamment à partir de l'hydrolysat résultat de l'étape/module d'hydrolyse thermique.

De préférence également, le procédé ou le système selon l'invention comprend au moins au moins une étape/module de récupération de phosphore à partir de l'eau de process.

De préférence, le procédé ou le système selon l'invention comprend au moins au moins une étape/module de récupération d'ammonium à partir de l'eau de process.

De préférence, l'eau de process est microfiltrée avant l'étape/module de récupération du phosphore et/ou de l'ammonium.

La personne du métier connait de nombreux procédés de récupération du phosphore et de l'ammonium.

De préférence, le phosphore est récupéré par un procédé chimique ou électrochimique. La récupération électrochimique du phosphore se fait notamment en précipitant le phosphate et l'ammonium grâce à une électrode de magnésium qui produit un précipité de magnésium-ammonium-phosphate. La récupération chimique du phosphore se fait notamment en produisant des sels de phosphate, en ajoutant par exemple au milieu à partir duquel on veut récupérer le phosphore du magnésium, du calcium et/ou du potassium), puis en cristallisant les sels à des valeurs de pH supérieures à 8,5.

De préférence, on applique la récupération électrochimique du phosphore préalablement à la digestion anaérobie principale, notamment à partir de l'hydrolysat résultat de l'étape/module d'hydrolyse thermique.

De préférence, on applique la récupération chimique du phosphore à l'eau de process.

De préférence, on récupère l'ammonium à partir de l'eau de process.

L'invention sera davantage exposée de manière non limitative, à l'aide du mode de réalisation préféré et de l'exemple qui suivent.

Un mode de réalisation préféré d'un procédé ou d'un système selon l'invention est représenté dans la Figure 1.

Au moins un intrant (1) comprenant de la matière organique, notamment des boues de station d'épuration et/ou de la fraction organique récupérable d'ordures ménagères, est digéré dans un digesteur anaérobie (2) pour donner un digestat (3) et un biogaz (4), en particulier du méthane.

Le digestat (3) est traité par une étape ou un module de carbonisation hydrothermale (5) qui produit un hydrochar (6) et de l'eau de process (7).

L'hydrochar (6) est traité par des étapes ou des moyens (8, 10), notamment une étape/un module de gazéification (8), qui produit un syngaz (9), et une étape/un module de méthanation (10), qui génèrent du biogaz (4).

Préalablement à sa digestion dans le digesteur anaérobie (2) l'intrant (1) peut être traité par une étape/un module d'hydrolyse thermique (11), éventuellement après avoir été épaissi par une étape/un module d'épaississement (14).

Par ailleurs, l'eau de process (7) peut être digérée par un deuxième digesteur anaérobie (12) pour générer du biogaz (4), en particulier du méthane.

Des étapes/modules de récupération de nutriments (13a, 13b), notamment le phosphore et l'ammonium, peuvent être présents. En particulier, une étape/un module de récupération de phosphore (13a) peut être présent pour récupérer du phosphore à partir de l'intrant (1), notamment traité par hydrolyse thermique (11), avant la première digestion anaérobie (2). Au moins une étape/un module de récupération de phosphore et/ou d'ammonium (13b) peut être présent pour récupérer du phosphore et/ou de l'ammonium à partir de l'eau de process (7) avant la deuxième digestion anaérobie (12).

Enfin, le biogaz (4), notamment le méthane, produit par le procédé/système selon l'invention, peut être collecté dans un module de gestion du biogaz (15).

### Exemple

Les inventeurs ont réalisé un modèle du système selon l'invention, avec l'agencement de modules décrit dans la figure 1 afin de quantifier la production de méthane obtenue à partir de l'hydrochar issu de la carbonisation hydrothermale.

Préalablement à la gazéification il est préférable que l'hydrochar soit séché pour atteindre environ 90% de masse sèche.

Le module de gazéification se compose d'un réacteur de gazéification avec dosage de combustible, de composants associés pour l'alimentation en fluides, d'un refroidisseur de gaz d'échappement en aval ainsi que d'un système de contrôle.

On prévoit une puissance nominale du module de gazéification de 30 kW. Cela correspond approximativement à une quantité d'hydrocharséché de 6 kg/h et à une quantité de gaz de synthèse (syngaz) produit de 5,3 Nm³/h. La teneur en hydrogène (H₂) du syngaz est estimée à environ 32 % (v/v).

Optionnellement, le syngaz est traité par une réaction du gaz à l'eau, ou réaction de « *water-shift* », ce qui a pour effet d'augmente la teneur en hydrogène (H₂) du syngaz en faisant réagir le monoxyde de carbone (CO) et l'eau (H₂O) pour produire du dioxyde de carbone (CO₂) et de l'hydrogène (H₂).

Le module de *water-shift* consiste en un réacteur cylindrique, disposé en une chambre de réaction verticale remplie de garnissages. La température est maintenue entre 300 et 500°C à l'aide d'un chauffage électrique couplé à un manteau permettant le refroidissement. Le réacteur est chauffé jusqu'à la température requise grâce à un dispositif de sondes de température électrique. Pour le refroidissement, de l'air transite à travers les deux parois du réacteur à l'aide d'un ventilateur. La température est régulée en fonction du taux de renouvellement d'airdans le dispositif. Le syngaz en entrée est préchauffé et alimente le réacteur de water-shift. L'eau nécessaire à la réaction est ajoutée au gaz sous forme de vapeur. La vapeur est produite au moyen d'un générateur de vapeur indépendant. A la sortie du réacteur, le gaz produit est refroidi avant d'entrer dans le module suivant. Pour cela, un échangeur air-eau avec séparation des condensats est utilisé. La température est régulée en fonction du débit d'eau utilisé. Lors de la mise en charge et l'arrêt du processus, un gaz d'inertage est utilisé.

Ce module permet d'augmenter la concentration d'hydrogène (H₂) du syngaz à la sortie du réacteur à environ 54 % (v/v).

Le syngaz est ensuite traité par un module de méthanation. On utilise un réacteur à lit de ruissellement pour la méthanisation biologique du H₂ et du CO₂ contenu dans le syngaz.

Le réacteur peut être un conteneur dont les parois résistent à la méthanation rempli de matériau d'emballage qui sert de surface de croissance et de fixation de la biomasse responsable du processus biologique. L'eau de traitement ruisselle continuellement pour humidifier le biofilm et lui fournir des nutriments. Le réacteur est gazéifié avec du syngaz à contre-courant du système de pulvérisation d'eau.

Le réacteur est adapté à un fonctionnement en continu et une quantité de gaz de 7,5 Nm³/h. Un enrichissement en méthane (CH₄) du syngaz de 1,6 % (v/v) de à environ 27 % (v/v) est obtenu. Cela correspond à une production de méthane d'environ 0,9 Nm³ CH₄/h.

Le rendement obtenu est d'environ 180 Nm³ CH₄ par tonne d'hydrochar sec, ou environ 145 Nm³ CH₄ par tonne de digestat sec, ou environ 88 Nm³ CH₄ par tonne d'intrant sec.

## Revendications

1. Procédé de production de biogaz et de traitement d'au moins un intrant contenant de la matière organique, comprenant les étapes suivantes :
- digérer de manière anaérobie au moins intrant contenant de la matière organique pour produire du méthane et un digestat ;
- réaliser une carbonisation hydrothermale du digestat pour produire de l'hydrochar et de l'eau de process ;
**caractérisé en ce que** :
dans une étape additionnelle, du méthane est généré à partir de la gazéification de l'hydrochar.

2. Procédé selon la revendication 1, dans lequel l'intrant comprend des boues de station d'épuration et/ou de la fraction organique récupérable d'ordures ménagères.

3. Procédé selon la revendication 1 ou 2, dans l'intrant est un mélange de boues de station d'épuration et de la fraction organique récupérable d'ordures ménagères.

4. Procédé selon l'une quelconque des revendications 1 à 3, dans lequel la digestion anaérobie est effectuée par un digesteur anaérobie sélectionné dans le groupe constitué d'un digesteur à piston, d'un digesteur par percolation et d'un digesteur à haute charge.

5. Procédé selon l'une quelconque des revendications 1 à 4, dans lequel la digestion anaérobie est effectuée par un digesteur à piston.

6. Procédé selon l'une quelconque des étapes 1 à 5, comprenant également une étape d'hydrolyse thermique préalable à la digestion anaérobie.

7. Procédé selon l'une quelconque des revendications 1 à 6, comprenant également une étape de digestion anaérobie de l'eau de process pour produire du méthane.

8. Procédé selon l'une quelconque des revendications 1 à 7, comprenant également au moins une étape de récupération de phosphore et/ou d'ammonium.

9. Système de production de biogaz et de traitement d'au moins un intrant comprenant de la matière organique, comprenant :
- un digesteur anaérobie, alimenté par au moins un intrant comprenant de la matière organique, qui produit du méthane et un digestat ;
- un module de carbonisation hydrothermale qui produit un hydrochar et de l'eau de process à partir du digestat ;
**caractérisé en ce que**:
le système comprend en outre des moyens pour générer du méthane à partir de l'hydrochar.

10. Système selon la revendication 9, dans lequel les moyens pour générer du méthane à partir de l'hydrochar comprennent au moins un module de gazéification pour produire un syngaz comprenant du dioxyde carbone et/ou du monoxyde de carbone et du dihydrogène à partir de l'hydrochar et un module de méthanation pour produire du méthane à partir du dioxyde de carbone et/ou du monoxyde de carbone et du dihydrogène du syngaz.

11. Système selon la revendication 9 ou 10, dans lequel l'intrant comprend des boues de station d'épuration et/ou de la fraction organique récupérable d'ordures ménagères.

12. Système selon l'une quelconque des revendications 9 à 11, dans lequel le système est alimenté par un mélange de boues de station d'épuration et de la fraction organique récupérable d'ordures ménagères.

13. Système selon l'une quelconque des revendications 9 à 12, dans lequel le digesteur anaérobie est sélectionné dans le groupe constitué d'un digesteur à piston, d'un digesteur par percolation et d'un digesteur à haute charge.

14. Système selon l'une quelconque des revendications 9 à 13, dans lequel le digesteur anaérobie est un digesteur à piston.

15. Système selon l'une quelconque des revendications 9 à 14, comprenant un module d'hydrolyse thermique en amont du digesteur anaérobie.

16. Système selon l'une quelconque des revendications 9 à 15, comprenant un deuxième digesteur anaérobie produisant du méthane à partir de l'eau de process.

17. Système selon l'une quelconque des revendications 9 à 16, comprenant au moins un module de récupération de phosphore et/ou d'ammonium.
